(19) Europäisches Patentamt / European Patent Office / Office européen des brevets

(11) **EP 4 343 789 A1**

(12) **EUROPEAN PATENT APPLICATION**

(43) Date of publication:
**27.03.2024 Bulletin 2024/13**

(21) Application number: **22197496.7**

(22) Date of filing: **23.09.2022**

(51) International Patent Classification (IPC):
**G16H 50/30** (2018.01)    **G16H 50/70** (2018.01)
**A61B 5/00** (2006.01)    **G16H 10/60** (2018.01)
**G16H 40/63** (2018.01)    **G16H 40/20** (2018.01)

(52) Cooperative Patent Classification (CPC):
**G16H 50/70; G16H 50/30;** A61B 5/7275;
G16H 10/60; G16H 40/20; G16H 40/63

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WIRTH, Christoph Tobias**
  **Eindhoven (NL)**
• **PAUWS, Steffen Clarence**
  **Eindhoven (NL)**
• **SOKORELI, Ioanna**
  **5656AG Eindhoven (NL)**

(74) Representative: **Philips Intellectual Property & Standards**
**High Tech Campus 52**
**5656 AG Eindhoven (NL)**

(54) **METHODS AND SYSTEMS FOR PATIENT DISCHARGE OPTIMIZATION USING UPLIFT PROBABILITY**

(57)    A method (100) for determining a home care uplift probability for a hospitalized patient, comprising: (i) receiving (120) patient data from a medical record database; (ii) extracting (130) an uplift probability feature set from the received patient data; (iii) analyzing (140), at a first time point, the uplift probability feature set by a trained uplift probability algorithm (263) to determine an uplift probability for discharge of the hospitalized patient versus continued hospitalization; (iv) comparing (150) the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold; and (v) providing (160), via a user interface, the recommendation to a user.

FIG. 1

EP 4 343 789 A1

**Description**

BACKGROUND

Hospital stays can have serious complications for patients as a result of negative events such as hospital-acquired infections, falls, delirium, and depression, making a stay in the hospital even longer. Complications can also arise because of side-effects of in-hospital treatment. All these in-hospital complications can result in an about 10% rise in total inpatient cost.

As one non-limiting example, heart failure is a serious health issue affecting around 64.3 million people worldwide, with about 15 million cases in Europe and 6.2 million cases in the U.S. Heart failure is a chronic condition which is associated with a decreased quality of life, frequent hospital stays due to acute episodes of decompensation and high mortality. The median length of stay for an acute heart failure admission ranges from 2 to 6 days in the U.S. However, in the EU the mean length of stay is even longer with a mean duration of 9.5 days. A total of 23% of patients are readmitted to the hospital within the first 30 days after discharge. Approximately half of the patients will be admitted at least once within one year after diagnosis, 20% will be readmitted again within that same year, and over 80% will be readmitted within five years. Heart failure hospitalizations represent 1% to 2% of all hospital admissions and heart failure is the most common diagnosis in hospitalized patients older than 65 years. Readmissions can be caused by early discharge, inadequate patient education, insufficient post-discharge follow-up, and the intrinsic elusiveness of the disease. This has a detrimental impact on both patient burden and societal healthcare cost. Excessive readmissions in the U.S. are penalized by Medicare, where the average penalty can be 0.64% reduction in payment for each Medicare patient stay. The fines involved can be heavy for hospitals.

Accordingly, as a safe alternative for a hospitalization due to an acute episode, hospital systems explore the opportunity of care virtualization. Instead of staying in the hospital, patients are discharged and receive care at home using digital virtual care support such as remote patient management (RPM). Hospitals therefore aim at discharging patients earlier, but within safe bounds and with post-discharge home care support, in order to reduce inpatient costs and avoid hospital-acquired complications. However, these existing systems utilize static, outdated measurements and checklists when determining discharge and readmission probabilities.

SUMMARY OF THE DISCLOSURE

Accordingly, there is a continued need for systems and methods that more accurately predict the benefit of discharging a hospitalized patient for home care. Various embodiments and implementations herein are directed to a method and system configured to determine a home care uplift probability for a hospitalized patient using a home care uplift probability system. The system receives patient data from a medical record database and extracts an uplift probability feature set from the data. The system then analyzes that uplift probability feature set by a trained uplift probability algorithm to determine an uplift probability for discharge of the hospitalized patient versus continued hospitalization. The system compares the determined uplift probability to a predetermined uplift probability threshold to determine a recommendation, and provides the recommendation to a user.

Generally, in one aspect, a method for determining a home care uplift probability for a hospitalized patient is provided. The method includes: (i) receiving patient data from a medical record database; (ii) extracting an uplift probability feature set from the received patient data; (iii) analyzing, at a first time point, the uplift probability feature set by a trained uplift probability algorithm to determine an uplift probability for discharge of the hospitalized patient versus continued hospitalization; (iv) comparing the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold; and (v) providing, via a user interface, the recommendation to a user.

According to an embodiment, the method further includes repeating at least the analyzing and comparing steps for a second time point; and generating an average uplift probability from the determine uplift probabilities.

According to an embodiment, providing further comprises providing one or more of patient information and the determined uplift probability to the user via the user interface.

According to an embodiment, the first time point is triggered by a care event for the hospitalized patient. According to an embodiment, the first time point is a predetermined time point at a particular time during the hospitalized patient's stay.

According to an embodiment, the recommendation is provided via a user interface of a clinical decision support system.

According to an embodiment, the recommendation is provided via a user interface of a mobile device.

According to an embodiment, the method further includes receiving, from a clinician via the user interface, a decision regarding the provided recommendation; and implementing, by the clinician, the provided recommendation. According to an embodiment, the provided recommendation is a recommendation to discharge the hospitalized patient.

According to another aspect, a system for determining a home care uplift probability for a hospitalized patient is provided. The system includes: a trained uplift probability algorithm trained to analyze an uplift probability feature set for the hospitalized patient to determine an uplift probability for discharge of the hospitalized patient versus continued hospitalization; a user interface; and a processor. The processor is configured to: (i) receive patient data from a medical record database; (ii) extract an uplift probability feature set from the received patient data; (iii) analyze, at a first time point, the uplift probability feature set by the trained uplift probability algorithm to determine an uplift probability for discharge of the hospitalized patient versus continued hospitalization; (iv) compare the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold; and provide, via the user interface, the recommendation to a user.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.

FIG. 1 is a flowchart of a method for determining a home care uplift probability, in accordance with an embodiment.
FIG. 2 is a schematic representation of a home care uplift probability system, in accordance with an embodiment.
FIG. 3 is a flowchart of uplift probability determination time points, in accordance with an embodiment.
FIG. 4 is a flowchart of a method for determining a home care uplift probability, in accordance with an embodiment.
FIG. 5 is flowchart of a method for training an uplift probability algorithm, in accordance with an embodiment.
FIG. 6 is a graph of uplift probability determination time points, in accordance with an embodiment.

DETAILED DESCRIPTION OF EMBODIMENTS

The present disclosure describes various embodiments of a system and method configured to generate and provide an uplift probability for discharge to home care for a hospitalized patient. More generally, Applicant has recognized and appreciated that it would be beneficial to determine when to discharge a hospitalized patient to managed home care. Accordingly, a home care uplift probability system receives patient data from a medical record database and extracts an uplift probability feature set from the data. The system then analyzes that uplift probability feature set by a trained uplift probability algorithm to determine an uplift probability for discharge of the hospitalized patient versus continued hospitalization. The system compares the determined uplift probability to a predetermined uplift probability threshold to determine a recommendation, and provides the recommendation to a user. Note that in addition to discharging a patient to home care such as managed home care, a clinician can also decide to transfer a patient to a skilled nurse facility or a rehab program, such as with a form of care virtualization by remote patient monitoring (RPM).

According to an embodiment, the systems and methods described or otherwise envisioned herein can, in some non-limiting embodiments, be implemented as an element for a commercial product for patient analysis or monitoring, such as the Philips® Patient Flow Capacity Suite (PFCS) (available from Koninklijke Philips NV, the Netherlands), or any suitable system. For example, the system and method can be implemented within existing or future clinical decision support systems (CDS), applications, and devices. However, the disclosure is not limited to these devices or systems, and thus disclosure and embodiments disclosed herein can encompass any device or system capable of generating and reporting an uplift probability for a patient.

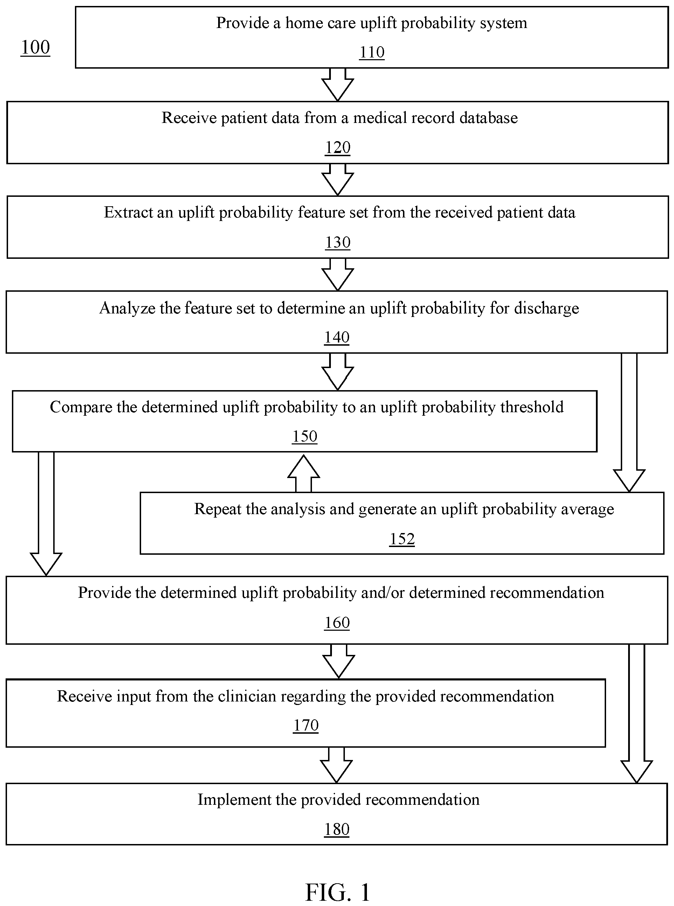

Referring to FIG. 1, in one embodiment, is a flowchart of a method 100 for determining a home care uplift probability for a hospitalized patient using a home care uplift probability system 200. The methods described in connection with the figures are provided as examples only, and shall be understood not limit the scope of the disclosure. The home care uplift probability system can be any of the systems described or otherwise envisioned herein. The home care uplift probability system can be a single system or multiple different systems.

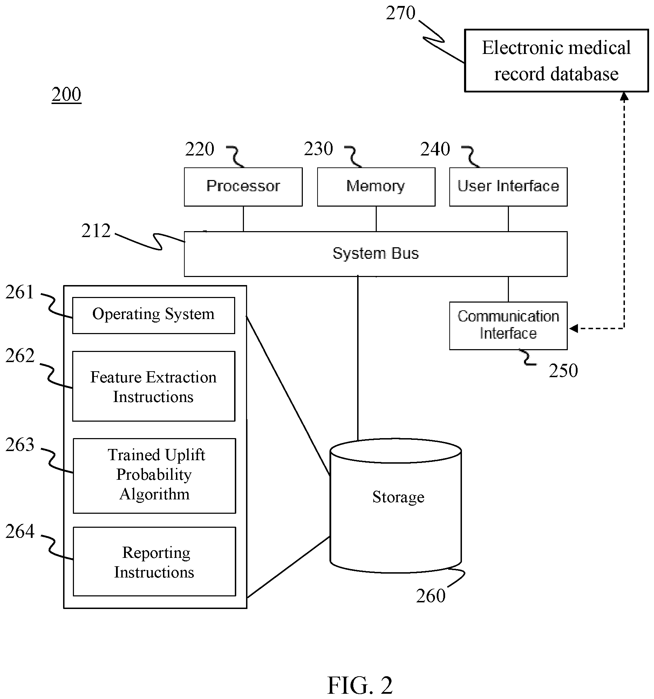

At step 110 of the method, a home care uplift probability system 200 is provided. Referring to an embodiment of a home care uplift probability system 200 as depicted in FIG. 2, for example, the system comprises one or more of a processor 220, memory 230, user interface 240, communications interface 250, and storage 260, interconnected via one or more system buses 212. It will be understood that FIG. 2 constitutes, in some respects, an abstraction and that the actual organization of the components of the system 200 may be different and more complex than illustrated. Additionally, home care uplift probability system 200 can be any of the systems described or otherwise envisioned herein. Other elements and components of home care uplift probability system 200 are disclosed and/or envisioned elsewhere herein.

At step 120 of the method, the home care uplift probability system receives or obtains patient data from a medical record database 270. The patient data can be any information about the patient that the home care uplift probability system can or may utilize for analysis as described or otherwise envisioned herein. According to an embodiment, the patient data comprises one or more of demographic information about the patient, a diagnosis for the patient, medical history of the patient, and/or any other information. For example, demographic information may comprise information about the patient such as name, age, body mass index (BMI), and any other demographic information. The diagnosis for the patient may be any information about a medical diagnosis for the patient, historical and/or current. The medical history of the patient may be any historical admittance or discharge information, historical treatment information, historical diagnosis information, historical exam or imaging information, and/or any other information.

Further non-limiting examples of patient data include psycho-social variables of the patient regarding things such as social support, living situation, smoking, alcohol consumption, depression, anxiety; functional variables of the patient regarding things such as frailty, dementia, selfefficacy, and motivation; signs and heart failure (HF) symptoms such as shortness of breath, fatigue, edema in ankles, legs and abdomen, ability to exercise, cough, and palpitations; type and number (for example measured by the Charlson Comorbidity Index (CCI)) of co-morbidities of the patient (e.g. diabetes, COPD, etc.); history of HF and other cardiac conditions of the patient like previous myocardial infarction (MI), and arrythmia; health utilization: the number of prior hospitalizations or emergency department (ED) visits; severity of heart failure - NYHA class (New York Heart Association) preferably assessed in multiple times; echo or radiological assessment of the heart such as ejection fraction levels, left atrial volume index (lavi), Right atrial pressure (rap), and pulmonary congestion; vital signs resulting from physical examination, such as blood pressure, heart rate, respiratory rate, weight, saturation, BMI; relevant blood test such as electrolytes (sodium, potassium), cholesterol, albumin, BUN, creatinine, and biomarkers like NTproBNP, ST2, hs-cTnT, and troponin; data on the current hospitalization such as days in ICU, days on wards, etc.; and medication and dosage for diuretics, ARBs, ACE inhibitors, beta blockers, and much more.

The patient data may be received or obtained from one or a plurality of different sources. According to an embodiment, the patient data is received from, retrieved from, or otherwise obtained from an electronic medical record (EMR) database or system 270. The EMR database or system may be local or remote. The EMR database or system may be a component of the home care uplift probability system, or may be in local and/or remote communication with the home care uplift probability system. The received patient data may be utilized immediately, or may be stored in local or remote storage for use in further steps of the method.

The patient data can include patient information and medical records that cover a period of time. For example, the patient data can include patient information and medical records for an entirety of a patient's hospitalization to date, for a predetermined previous time, or any other period of time. The patient data can be continuously received or obtained, or the patient data can be periodically received or obtained such as once an hour, once a day, and so on. Additionally or alternatively, the patient data can be received or obtained in response to a triggering event, such as a patient care event like availability of a new record, transitioning to a new diagnosis or care routine, moving to a new location, and so on. Many other triggering events are possible.

At step 130 of the method, the home care uplift probability system analyzes some or all of the received patient data to extract an uplift probability feature set (optionally denoted here as X) for the patient. The uplift probability feature set X can include any patient information that the home care uplift probability system can or may utilize for analysis as described or otherwise envisioned herein. The feature set $X$ can include, for example, various EMR or hospital-based data elements captured at various moment in time from admission to discharge. Some data elements will change values when captured repeatedly over time, and others will have constant values.

Each of the plurality of features in the uplift probability feature set $X$ can be identified or extracted using known methods for identifying and extracting features in patient data. According to an embodiment, the plurality of features utilized by the home care uplift probability system for analysis may depend on the patient's diagnosis, treatment, demographics, and/or any other information specific to the patient. The parameters of feature set $X$ can be determined based in part or in whole on user input, or can be identified or determined in part or in whole from the received or obtained patient data. Once extracted, feature set X can be utilized immediately, or may be stored in local or remote storage for use in further steps of the method.

At step 140 of the method, a trained uplift probability algorithm of the home care uplift probability system analyzes the uplift probability feature set X at a first time point, in order to determine an uplift probability for discharge of the hospitalized patient versus continued hospitalization. The trained uplift probability algorithm can be any algorithm or model configured to utilize the uplift probability feature set X as an input to generate an uplift probability for discharge of the hospitalized patient versus continued hospitalization.

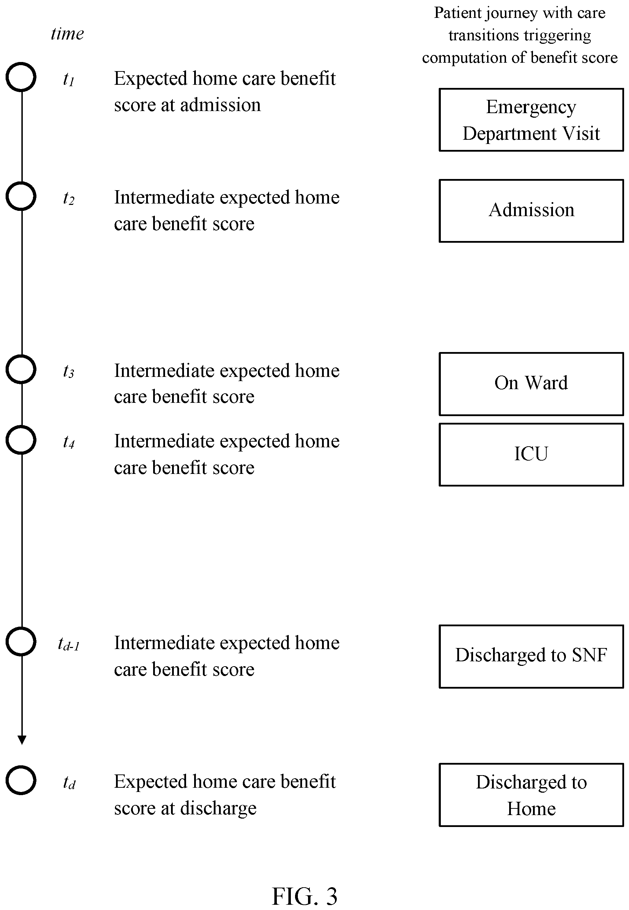

According to an embodiment, the trained uplift probability algorithm analyzes the uplift probability feature set X at any one or more of a plurality of different time points. For example, the generated uplift probability for discharge of the hospitalized patient versus continued hospitalization, also called a "benefit score," can be measured or determined at several inpatient moments of time from admission time to actual discharge, as shown in FIG. 3. This score, which can be offered as a service to recommend for a patient transition to the clinical staff, expresses the expected benefit (as an uplift probability) to discharge of a patient with home care support in comparison to a continued hospitalization. In FIG. 3, for example, there is an initial home benefit score calculation ($t_1$), a discharge home benefit score calculation ($t_d$), and several intermediate home benefit score calculations ($t_2$, $t_3$, $t_4$, $t_{d-1}$). Many other combinations are possible.

According to an embodiment, the uplift probability is calculated at some point (predetermined, random, or in response to a triggering event) every day during the hospital stay of the patient, from the day of admission to the end of the patient's stay. The trend of this uplift would also provide information on the progression of the health status of the patient. For example, is the patient getting more stable over time? Or, how many days must the patient continue to stay in the hospital? This information can be provided to the clinician as described or otherwise envisioned herein.

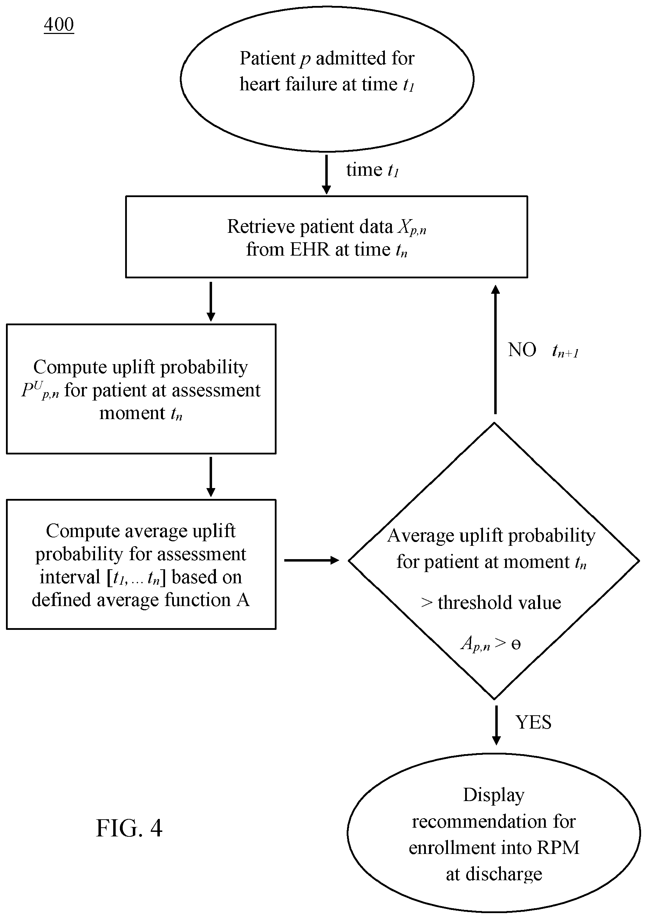

Referring to FIG. 4, in one embodiment, is a flowchart of a method 400 for generating and providing an uplift probability for discharge to a clinician. According to an embodiment, after a patient is admitted for acute heart failure (HF), the patient's uplift probability $P^U$ is calculated at various assessment moments $t_n$. Assessment moments can be triggered by transitions to a different care setting (e.g. ED, ICU, ward) or by ordered measurements (e.g. lab test, vital signs) or updates in medication or performed medical exams. Assessment moments can also follow a regular frequency (e.g. daily) independent of new patient-level data entry into the EHR. Every assessment moment is a call to the CDS service and data required for computation is fetched from the EHR. According to an embodiment, the uplift probability is defined as the difference between success probabilities in the case the patient received treatment (enrolled into an out-of-hospital RPM program) or did not (control group - continued hospitalization). According to an embodiment, success can be measured against a selected outcome, for example avoidance of adverse events such as readmission or complications in post-acute care after discharge, among other selected or predetermined or identified outcomes.

According to an embodiment, the uplift probability can be derived from an uplift model defined using the following equation:

$$P^U(X) := P^T(Y = 1|X) - P^C(Y = 1|X) \qquad \text{Eq. 1}$$

where $Y = 1$ denotes the desired (or positive, success) outcome of non-occurrence of adverse events (e.g. no readmission, no death) and $Y = 0$ the respective classification for an undesirable (or negative, failure) outcome (e.g. readmission occurred, death). $P^T$ and $P^C$ denote the probability distributions of the outcome Y estimated on patients in the treated (received an RPM program) and control group respectively; $P^{C,T}(Y = 1|X)$ is the class probability of a positive outcome conditional on the feature vector X. According to an embodiment, the uplift model is built and validated in an initial trial or pilot phase which requires a defined volume of patients to receive the RPM home care intervention after a discharge for HF and compare that to a matched control group who did not receive the treatment - but stayed in the hospital. The outcome variable is then evaluated after a defined period (e.g. 30 days, one year). According to another embodiment, outcomes can also be defined for a continuous variable L (e.g. hospital-free days alive after index admission) when its desired outcome value is larger than a pre-defined threshold value M, for example the sample's median value.

The uplift model's binary class variable Y can then be obtained by the following discretizing function:

$$Y = \begin{cases} 1, & \text{if } L > M \\ 0, & \text{otherwise} \end{cases} \qquad \text{(Eq. 2)}$$

An uplift model can be intuitively built from two models, for example by logistic regression, to each estimate the probabilities $P^T$ and $P^C$ and then subtract these to estimate the uplift $P^U$.

In another embodiment, the outcomes variable can be transformed by the following function (see TABLE 1):

$$Z = \begin{cases} 1, \text{ if } Group = T \cap Y = 1 \\ 1, \text{ if } Group = C \cap Y = 0 \\ 0, \qquad \text{otherwise} \end{cases} \qquad \text{(Eq. 3)}$$

and the uplift probability can be computed based on the following single-model approach:

$$P^U(X) = 2Pr(Z = 1|X) - 1 \qquad \text{(Eq. 4)}$$

where Pr can be estimated for example by on logistic regression model with the transformed outcomes variable Z. The model requires equal group sizes, this however can always be achieved be re-weighting the groups. The advantage of a single uplift model approach is that it predicts the uplift directly and not as a difference in the case of the two-model approach; also the single model is built from a larger sample size of both the treated and control cohort of the pilot phase. In the two-model construction, the treatment model and the control model are built independently and focus on predicting the outcome separately. This separation could also result in having different set of features X for each model. Each model can prioritize different features for having high predictive power to estimate the outcome while disregarding the features that best estimate the uplift across two models.

TABLE 1. Variable Transformation.

|  | Y = 1 (e.g. no readmission) | Y = 0 (e.g. readmission occurred) |
|---|---|---|
| Group = T | Z = 1 | Z = 0 |
| (Treated) |  |  |
| Group = C (Control) | Z = 0 | Z = 1 |

For a patient p, uplift probabilities $P^U_{p,i}$ can be computed for each assessment moment $t_i$ where the uplift model $P^U$ is evaluated with the current patient-specific feature vector $x_{p,i}$ by:

$$P^U_{p,i} := P^U\left(X = x_{p,i}\right) \qquad \text{(Eq. 5)}$$

Once a series of uplift probabilities $P^U_{p,i}$ for a patient is computed over an assessment interval [$t_i$, ..., $t_n$] an averaging function is chosen (e.g., moving average, weighted means) to compute the average value $A_{p,n}$ at the most current assessment moment $t_n$. If $A_{p,n} > \theta$ where $\theta \geq 0$ is a threshold value, the patient is recommended for discharge with enrollment into an RPM home care program.

According to an embodiment, the value of $\theta$ can be determined based on resource constraints such as budget limit, RPM staff availability, etc. With a $\theta > 0$ not all patients with a positive uplift probability will be recommended for an RPM program though their expected benefit is positive. $\theta$ could take a fixed value defined at the beginning of a program or can be a variable continuously changing according to the resource constraints. Methods for capacity management of RPM resource allocation based on human-determined or predictive modeling could be utilized to optimize the threshold based on available resources and to optimize capacity management.

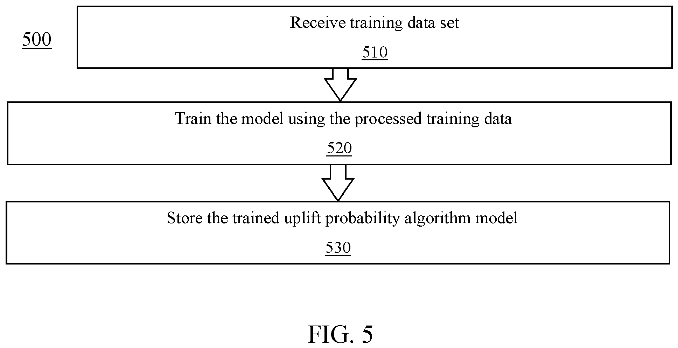

Referring to FIG. 5, in one embodiment, is a flowchart of a method 500 for training the uplift probability algorithm of the home care uplift probability system 200. At step 510 of the method, the system receives or obtains a training data set comprising training data for a plurality of patients, such as historical patient data and information. The training data can comprise input such as demographic information about the patients, diagnoses for the patients, medical history of the patients, and/or any other information. For example, demographic information may comprise information about the patients such as name, age, body mass index (BMI), and any other demographic information. The diagnosis for the patients may be any information about a medical diagnosis for the patients, historical and/or current. The medical history of the patients may be any historical admittance or discharge information, historical treatment information, historical diagnosis information, historical exam or imaging information, and/or any other information. The training data may be stored in and/or received from one or more databases. The database may be a local and/or remote database. For example, the home care uplift probability system may comprise a database of training data.

According to an embodiment, the home care uplift probability system may comprise a data pre-processor or similar component or algorithm configured to process the received training data. For example, the data pre-processor analyzes the training data to remove noise, bias, errors, and other potential issues. The data pre-processor may also analyze the input data to remove low-quality data. Many other forms of data pre-processing or data point identification and/or extraction are possible.

At step 520 of the method, the system trains the machine learning algorithm, which will be the algorithm utilized in analyzing the input information as described or otherwise envisioned. The machine learning algorithm is trained using the training data set according to known methods for training a machine learning algorithm. According to an embodiment, the algorithm is trained, using the processed training dataset, to utilize an uplift probability feature *set X to* determine an uplift probability for discharge of the hospitalized patient versus continued hospitalization as described or otherwise envisioned herein.

At step 630 of the method, the trained uplift probability algorithm of the home care uplift probability system is stored for future use. According to an embodiment, the model may be stored in local or remote storage.

Returning to method 100 in FIG. 1, at step 150 of the method, the home care uplift probability system compares the determined uplift probability to a predetermined uplift probability threshold to determine a recommendation. According to an embodiment, the recommendation can be to keep the hospitalized patient hospitalized when the determined uplift probability is below the predetermined uplift probability threshold. According to an embodiment, the recommendation can be to discharge the hospitalized patient when the determined uplift probability is above the predetermined uplift probability threshold. Other recommendations are possible.

According to an embodiment, the threshold can be determined by a clinician, or can be determined by the system or another system. For example, according to an embodiment, the value of the threshold can be determined based on resource constraints such as budget limit, RPM staff availability, and so on. When the threshold is greater than zero, as it is expected to be, not all patients with a positive uplift probability will be recommended for an RPM program though their expected benefit is positive. The threshold could be a fixed value defined at the beginning of a program or can be a variable continuously changing according to the resource constraints. Methods for capacity management of RPM resource allocation based on human-determined or predictive modeling could be utilized to optimize the threshold based on available resources and to optimize capacity management.

According to an embodiment, the uplift probability can be determined for the patient at more than one time point. For example, referring to the non-limiting example shown in FIG. 3, there is an initial home benefit score calculation ($t_1$), a discharge home benefit score calculation($t_d$), and several intermediate home benefit score calculations ($t_2$, $t_3$, $t_4$, $t_{d-1}$). Many other combinations are possible.

According to an embodiment, for complex patients, the uplift probability might not pass the threshold at any time during the hospital stay. Since a patient cannot stay endlessly in a hospital, in this case a maximum length of stay can be incorporated for which a clinical decision needs to be made on the care transition. For example, rather than simply comparing the determined uplift probability to a predetermined threshold and making a determination, the system can compare the determined uplift probability to the predetermined threshold and, if it does not meet or exceed the threshold, can then compare the patient's current stay to a predetermined length of stay threshold, and provide a recommendation if the current stay meets or exceeds the predetermined length of stay threshold.

At optional step 152 of the method, the system generates an average uplift probability from two or more determined uplift probabilities. As described above, in accordance with an embodiment, once a series of uplift probabilities $P_{p,i}^U$ for a patient is computed over an assessment interval $[t_1, ..., t_n]$ an averaging function can be chosen (e.g., moving average, weighted means) to compute the average value $A_{p,n}$ at the most current assessment moment $t_n$. The system can then return to step 150 of the method and compare the average value $A_{p,n}$ to the threshold in order to generate a recommendation. For example, if $A_{p,n} > \theta$ (where $\theta \geq 0$), the patient is recommended for discharge with enrollment into an RPM home care program. If $A_{p,n} < \theta$, the patient is recommended to remain hospitalized.

At step 160 of the method, the determined recommendation and/or the determined uplift probability for the patient is provided to a user via a user interface of the home care uplift probability system. The user can be any individual, although it will typically be a clinician involved in or responsible for care of the patient. The determined recommendation and/or the determined uplift probability may be provided to the user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. According to an embodiment, the display may further comprise one or more of the patient's name and/or a treatment recommendation, among many other types of information.

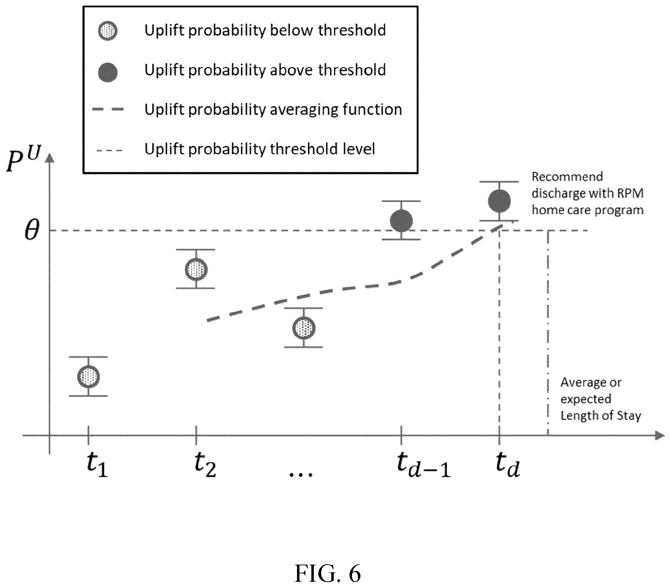

According to an embodiment, the determined recommendation and/or the determined uplift probability for the patient, optionally including other information, can be provided to a user via a variety of other mechanisms, devices, and systems. For example, in accordance with an embodiment, the information can be provided via a clinical decision support (CDS) device, system, or application. The information can be provided in the form of cards representing patient-specific recommendations that are presented to the user within the app which may be launched from within the CDS Client, e.g., an EHR system. The information presented to the user can contain the uplift probabilities $P^U$ for the patient to benefit from enrolment into a care program at the measurement times $t_n$ and its averaging function over the various measurement moments, and a recommendation if the patient would benefit from enrolment into a care program upon discharge at time $t_d$ given the uplift probability averaging function curve surpassed the threshold level Q. Referring to FIG. 6, in one embodiment, is a graph over time ($t_1$, $t_2$... $t_{d-1}$, $t_d$) with the uplift probabilities $P^U$ (i.e., the plotted data points) for the patient, along with the averaged uplift probability, both of which are compared to the predetermined uplift probability threshold level ($\theta$). The example visualization in FIG. 6 also shows the savings in time spent in the inpatient ward as the difference to the average or expected length of stay for that patient.

According to an embodiment, the information can be provided to the clinician via a clinical decision support service app. For example, the system and method can be implemented as an AI-based RPM Patient Recommendation as a Service solution which provides clinical decision support (CDS) for health care professionals or hospital staff identifying patients for enrollment into post-discharge care programs. The system provides a recommendation for a patient transition which is displayed via a clinician-facing CDS service app. The CDS service app will then display the recommendation, such as "discharge patient with RPM home care program." The user can then accept, reject, or overrule the recommendation displayed by the CDS service app. When an app user confirms a decision, the patient can be scheduled to receive transition services, which depending on the confirmed decision can invoke services for discharge of the patient for starting the onboarding process into a care management program including RPM services.

According to an embodiment, CDS service app users can be health care professionals or staff involved in examining a patient's readiness for discharge or a patient flow coordinator in the clinical command center (CLOC) or of a hospital's admission and discharge office. The CDS service app can be installed on smartphones, tablets, laptops, etc. The app can also be launched from within an CDS client, such as an EHR system, as an CDS service request using interoperable standards such as RESTful APIs and interaction patters with connected data systems described for example by CDS hooks. The CDS service can either initiated by an app user or activated by an automatic trigger, e.g. new data entry which calls the algorithm and produces an updated output.

At optional step 170 of the method, the home care uplift probability system receives, from a clinical via a user interface, a decision regarding the provided recommendation. For example, a **clinician or other decisionmaker utilizes the displayed recommendation to make a care decision for the patient. The decision can be input provided to the system by the clinician via the user interface, such as a selection of the recommendation, a rejection of the recommendation, a request for more information, and/or any other input. The input can be provided via any mechanism for providing information via a user interface. The user interface can be the user interface of the system, a mobile computing device, or any other user interface, and the input can be communicated to the home care uplift probability system via wired and/or wireless communication. Providing the decision or input regarding the recommendation can trigger a series of events that results in implementation of the recommendation, as described or otherwise envisioned herein.**

At optional step 180 of method 100 depicted in FIG. 1, the provided recommendation can be implemented, such as by the clinician and/or by a care system of the hospital or other care setting. For example, implementation can comprise a prescription, order, additional testing, and/or another implementation. According to an embodiment where the recommendation it to keep the hospitalized patient hospitalized when the determined uplift probability is below the predetermined uplift probability threshold, implementation of the recommendation can be an order to keep the patient hospitalized. According to an embodiment where the recommendation is to discharge the hospitalized patient when the determined uplift probability is above the predetermined uplift probability threshold, implementation of the recommendation can be an order to discharge the patient or to prepare the patient for discharge. **Many other implementations are possible.**

**Referring to FIG. 2 is a schematic representation of a home care uplift probability system 200.** System 200 may be any of the systems described or otherwise envisioned herein, and may comprise any of the components described or otherwise envisioned herein. It will be understood that FIG. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example LI, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the electronic medical record system 270 is an electronic medical records database from which the information about the patient, including patient data and/or training data, may be obtained or received. The electronic medical records database may be a local or remote database and is in direct and/or indirect communication with the home care uplift probability system 200. Thus, according to an embodiment, home care uplift probability system comprises an electronic medical record database or system 270.

According to an embodiment, storage 260 of system 200 may store one or more algorithms, modules, and/or instructions to carry out one or more functions or steps of the methods described or otherwise envisioned herein. For example, the system may comprise, among other instructions or data, feature extraction instructions 262, a trained uplift probability algorithm 263, and/or reporting instructions 264.

According to an embodiment, the feature extraction instructions 262 direct the system to analyze some or all of the received patient data to extract an uplift probability feature set X for the patient. The uplift probability feature set X can include any patient information that the home care uplift probability system can or may utilize for analysis as described or otherwise envisioned herein. The feature set X can include, for example, various EMR or hospital-based data elements captured at various moment in time from admission to discharge. Some data elements will change values when captured repeatedly over time, and others will have constant values. Each of the plurality of features in the uplift probability feature set X can be identified or extracted using known methods for identifying and extracting features in patient data. According to an embodiment, the plurality of features utilized by the home care uplift probability system for analysis may depend on the patient's diagnosis, treatment, demographics, and/or any other information specific to the patient. The parameters of feature set $X$ can be determined based in part or in whole on user input, or can be identified or determined in part or in whole from the received or obtained patient data. Once extracted, feature *set X can* be utilized immediately, or may be stored in local or remote storage for use in further steps of the method.

According to an embodiment, the trained uplift probability algorithm 263 is trained to analyze the uplift probability feature set *X* to determine an uplift probability for discharge of the hospitalized patient versus continued hospitalization. The trained uplift probability algorithm can be any algorithm or model configured to utilize the uplift probability feature set *X* as an input to generate an uplift probability for discharge of the hospitalized patient versus continued hospitalization. According to an embodiment, the trained uplift probability algorithm analyzes the uplift probability feature set *X* at any one or more of a plurality of different time points. Referring to FIG. 4 is a method for determining an uplift probability for discharge of a hospitalized patient using the trained uplift probability algorithm 263, and referring to FIG. 5 is a method for training an uplift probability algorithm 263.

According to an embodiment, the reporting instructions 264 direct the system to generate and provide to a user via a user interface the determined recommendation and/or the determined uplift probability for the patient. The user can be any individual, although it will typically be a clinician involved in or responsible for care of the patient. The determined recommendation and/or the determined uplift probability may be provided to the user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands. According to an embodiment, the display may further comprise one or more of the patient's name and/or a treatment recommendation, among many other types of information.

According to an embodiment, the determined recommendation and/or the determined uplift probability for the patient, optionally including other information, can be provided to a user via a variety of other mechanisms, devices, and systems. For example, in accordance with an embodiment, the information can be provided via a clinical decision support (CDS) device, system, or application. The information can be provided in the form of cards representing patient-specific recommendations that are presented to the user within the app which may be launched from within the CDS Client, e.g., an EHR system. According to an embodiment, the information can be provided to the clinician via a clinical decision support service app. For example, the system and method can be implemented as an AI-based RPM Patient Recommendation as a Service solution which provides clinical decision support (CDS) for health care professionals or hospital staff identifying patients for enrollment into post-discharge care programs. The system provides a recommendation for a patient transition which is displayed via a clinician-facing CDS service app. The CDS service app will then display the recommendation, such as "discharge patient with RPM home care program." The user can then accept, reject, or overrule the recommendation displayed by the CDS service app. When an app user confirms a decision, the patient can be scheduled to receive transition services, which depending on the confirmed decision can invoke services for discharge of the patient for starting the onboarding process into a care management program including RPM services. According to an embodiment, CDS service app users can be health care professionals or staff involved in examining a patient's readiness for discharge or a patient flow coordinator in the clinical command center (CLOC) or of a hospital's admission and discharge office. The CDS service app can be installed on smartphones, tablets, laptops, etc. The app can also be launched from within an CDS client, such as an EHR system, as an CDS service request using interoperable standards such as RESTful APIs and interaction patters with connected data systems described for example by CDS hooks. The CDS service can either initiated by an app user or activated by an automatic trigger, e.g. new data entry which calls the algorithm and produces an updated output.

Accordingly, within the context of the disclosure herein, aspects of the embodiments may take the form of a computer program product embodied in one or more non-transitory computer-readable media having computer readable program code embodied thereon. Thus, according to one embodiment is a non-transitory computer-readable storage medium comprising computer program code instructions which, when executed by a processor, enables the processor to carry out a method including: (i) receive patient data from a medical record database; (ii) extract an uplift probability feature set from the received patient data; (iii) analyze, at a first time point, the uplift probability feature set by the trained uplift probability algorithm to determine an uplift probability for discharge of the hospitalized patient versus continued hospitalization; (iv) compare the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold; and (v) provide, via a user interface, the recommendation to a user. The program code may perform entirely on a user's computer, partly on a user's computer, as a stand-alone software package, partly on a user's computer and partly on a remote computer, or entirely on a remote computer or server.

According to an embodiment, the home care uplift probability system is configured to process many thousands or millions of datapoints in the input data used to train the system, as well as to process and analyze the received patient data. For example, generating a functional and skilled trained system using an automated process such as feature identification and extraction and subsequent training requires processing of millions of datapoints from input data and the generated features. This can require millions or billions of calculations to generate a novel trained system from those millions of datapoints and millions or billions of calculations. As a result, the trained system is novel and distinct based on the input data and parameters of the machine learning algorithm, and thus improves the functioning of the home care uplift probability system. Generating a functional and skilled trained system comprises a process with a volume of calculation and analysis that a human brain cannot accomplish in a lifetime, or multiple lifetimes. By providing an improved patient analysis, this novel home care uplift probability system has an enormous positive effect on patient analysis and care compared to prior art systems.

There is no tool available to physicians that provides a continuous assessment of risk and enables them to make the right treatment choice at the earliest possible moment. Currently physicians assess the clinical status of the patient in a non-continuous manner and based on this assessment make a care decision. They also do not have quantitative information of the benefit of different care models for the patient. The methods and systems described or otherwise envisioned herein provide such a system and the necessary quantitative information.

Among other improvements, the home care uplift probability system allows hospitals to gain efficiency in terms of healthcare inpatient resource utilization by enabling earlier discharge of patients with continued coordinated care at home. Expensive inpatient days are substituted with days spent in a lower acuity setting. Consequently, the methods and systems described herein decrease the mean length of stay for the whole patient population.

The methods and systems also improve quality of life and patient satisfaction for patients and relatives by experiencing more care from the comfort of the home. The risk of hospital-acquired complications for elderly patients is lowered. For patients with acute heart failure, the risk of experiencing side-effects of the in-hospital treatment is lowered. These complications typically extend the hospital stay or even lead to in-hospital death.

The methods and systems are optimized for a desired outcome of no readmission and no death, or the number of hospital-free days alive after an index admission to reduce the pertaining risk. Any remaining risk of a readmission or death are mitigated by providing a patient-safe discharge moment, adequate patient education, and post-discharge follow-up by home care.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, fieldprogrammable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A method (100) for determining a home care uplift probability for a hospitalized patient, comprising:

   receiving (120) patient data from a medical record database;
   extracting (130) an uplift probability feature set from the received patient data;
   analyzing (140), at a first time point, the uplift probability feature set by a trained uplift probability algorithm (263) to determine an uplift probability for discharge of the hospitalized patient versus continued hospitalization;
   comparing (150) the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold;
   providing (160), via a user interface, the recommendation to a user.

2. The method of claim 1, further comprising:

   repeating at least the analyzing and comparing steps for a second time point; and
   generating (152) an average uplift probability from the determine uplift probabilities.

3. The method of claim 1, wherein providing further comprises providing one or more of patient information and the determined uplift probability to the user via the user interface.

4. The method of claim 1, wherein the first time point is triggered by a care event for the hospitalized patient.

5. The method of claim 1, wherein the first time point is a predetermined time point at a particular time during the hospitalized patient's stay.

6. The method of claim 1, wherein the recommendation is provided via a user interface of a clinical decision support system.

7. The method of claim 1, wherein the recommendation is provided via a user interface of a mobile device.

8. The method of claim 1, further comprising the steps of:

receiving (170), from a clinician via the user interface, a decision regarding the provided recommendation; and implementing (180), by the clinician, the provided recommendation.

9. The method of claim 8, wherein the provided recommendation is a recommendation to discharge the hospitalized patient.

10. A system (200) for determining a home care uplift probability for a hospitalized patient, comprising:

a trained uplift probability algorithm (263) trained to analyze an uplift probability feature set for the hospitalized patient to determine an uplift probability for discharge of the hospitalized patient versus continued hospitalization; a user interface (240); and a processor (220) configured to: (i) receive patient data from a medical record database (270); (ii) extract an uplift probability feature set from the received patient data; (iii) analyze, at a first time point, the uplift probability feature set by the trained uplift probability algorithm to determine an uplift probability for discharge of the hospitalized patient versus continued hospitalization; (iv) compare the determined uplift probability to a predetermined uplift probability threshold to determine a recommendation, wherein the recommendation is to keep the hospitalized patient hospitalized when the determined uplift probability is below the predetermined uplift probability threshold, and wherein the recommendation is to discharge the hospitalized patient when the determined uplift probability is above the predetermined uplift probability threshold; and provide, via the user interface, the recommendation to a user.

11. The system of claim 10, further comprising a medical record database (270) comprising the patient data.

12. The system of claim 10, wherein the processor is further configured to repeat at least the analyzing and comparing steps for a second time point; and further configured to generate an average uplift probability from the determine uplift probabilities.

13. The system of claim 10, wherein providing further comprises providing one or more of patient information and the determined uplift probability to the user via the user interface.

14. The system of claim 10, wherein the recommendation is provided via a user interface of a clinical decision support system.

15. The system of claim 10, wherein the recommendation is provided via a user interface of a mobile device.

100

| Provide a home care uplift probability system |
| :---: |
| 110 |

⇩

| Receive patient data from a medical record database |
| :---: |
| 120 |

⇩

| Extract an uplift probability feature set from the received patient data |
| :---: |
| 130 |

⇩

| Analyze the feature set to determine an uplift probability for discharge |
| :---: |
| 140 |

⇩

| Compare the determined uplift probability to an uplift probability threshold |
| :---: |
| 150 |

⇧

| Repeat the analysis and generate an uplift probability average |
| :---: |
| 152 |

⇩

| Provide the determined uplift probability and/or determined recommendation |
| :---: |
| 160 |

⇩

| Receive input from the clinician regarding the provided recommendation |
| :---: |
| 170 |

⇩

| Implement the provided recommendation |
| :---: |
| 180 |

## FIG. 1

FIG. 2

*time*

Patient journey with care
transitions triggering
computation of benefit score

$t_1$   Expected home care benefit score at admission

> Emergency Department Visit

$t_2$   Intermediate expected home care benefit score

> Admission

$t_3$   Intermediate expected home care benefit score

> On Ward

$t_4$   Intermediate expected home care benefit score

> ICU

$t_{d-1}$   Intermediate expected home care benefit score

> Discharged to SNF

$t_d$   Expected home care benefit score at discharge

> Discharged to Home

## FIG. 3

400

Patient $p$ admitted for heart failure at time $t_1$

time $t_1$

Retrieve patient data $X_{p,n}$ from EHR at time $t_n$

Compute uplift probability $P^U_{p,n}$ for patient at assessment moment $t_n$

Compute average uplift probability for assessment interval $[t_1, \ldots t_n]$ based on defined average function A

Average uplift probability for patient at moment $t_n$ > threshold value

$A_{p,n} > \theta$

NO $t_{n+1}$

YES

Display recommendation for enrollment into RPM at discharge

FIG. 4

| |
|---|
| Receive training data set<br>510 |

500

$\Downarrow$

| |
|---|
| Train the model using the processed training data<br>520 |

$\Downarrow$

| |
|---|
| Store the trained uplift probability algorithm model<br>530 |

FIG. 5

FIG. 6

Europäisches
Patentamt

European
Patent Office

Office européen
des brevets

## EUROPEAN SEARCH REPORT

Application Number

EP 22 19 7496

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/411193 A1 (WALD RANDALL [US] ET AL) 31 December 2020 (2020-12-31) * figures 1A-8C * * page 1, paragraph 0001 – paragraph 0012 * * page 3, paragraph 0020 – page 7, paragraph 0053 * * page 10, paragraph 0154 – page 11, paragraph 0163 * ----- | 1-15 | INV. G16H50/30 G16H50/70 ADD. A61B5/00 G16H10/60 G16H40/63 G16H40/20 |
| X | US 2021/098090 A1 (THOMAS BEX GEORGE [US] ET AL) 1 April 2021 (2021-04-01) * the whole document, in particular paragraphs [0001], [0032], [0042]-[0085], [0093]-[0127], and figures 1A and 1B * ----- | 1-15 | |
| X | CA 2 945 134 A1 (PARKLAND CENTER FOR CLINICAL INNOVATION [US]) 15 October 2015 (2015-10-15) * figures 1-16 * * page 1, paragraph 0001 – page 17, paragraph 0058 * * page 40, paragraph 00128 – page 42, paragraph 00136 * ----- | 1-15 | |
| A | Anonymous: "Proportionality (mathematics) – Wikipedia", , 1 September 2022 (2022-09-01), XP93026651, Retrieved from the Internet: URL:https://en.wikipedia.org/w/index.php?title=Proportionality_(mathematics)&oldid=1107973730 [retrieved on 2023-02-23] * the whole document * ----- | 1-15 | |

**TECHNICAL FIELDS SEARCHED (IPC)**

G16H
A61B

The present search report has been drawn up for all claims

| Place of search | Date of completion of the search | Examiner |
|---|---|---|
| Munich | 27 February 2023 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 7496

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2023

| Patent document cited in search report | | Publication date | Patent family member(s) | | Publication date |
|---|---|---|---|---|---|
| US 2020411193 | A1 | 31-12-2020 | US 2020411193 A1 | | 31-12-2020 |
| | | | US 2023019580 A1 | | 19-01-2023 |
| US 2021098090 | A1 | 01-04-2021 | NONE | | |
| CA 2945134 | A1 | 15-10-2015 | CA 2945131 A1 | | 15-10-2015 |
| | | | CA 2945134 A1 | | 15-10-2015 |
| | | | CA 2945136 A1 | | 15-10-2015 |
| | | | CA 2945137 A1 | | 15-10-2015 |
| | | | CA 2945138 A1 | | 15-10-2015 |
| | | | CA 2945143 A1 | | 15-10-2015 |
| | | | EP 3129945 A1 | | 15-02-2017 |
| | | | EP 3129949 A2 | | 15-02-2017 |
| | | | EP 3129950 A2 | | 15-02-2017 |
| | | | EP 3129951 A1 | | 15-02-2017 |
| | | | WO 2015157570 A1 | | 15-10-2015 |
| | | | WO 2015157572 A1 | | 15-10-2015 |
| | | | WO 2015157573 A2 | | 15-10-2015 |
| | | | WO 2015157575 A2 | | 15-10-2015 |
| | | | WO 2015157576 A1 | | 15-10-2015 |
| | | | WO 2015157577 A2 | | 15-10-2015 |

EPO FORM P0459

For more details about this annex : see Official Journal of the European Patent Office, No. 12/82